# EUROPÄISCHE PATENTANMELDUNG

(11) **EP 1 952 770 A1**
(43) Veröffentlichungstag der Anmeldung: **06.08.2008**
(21) Anmeldenummer: 07002451.8
(22) Anmeldetag: 05.02.2007
(51) Int. Cl.: A61B 17/12, A61B 17/30, A61B 17/42, A61F 6/20

(54) **Ringapplikator für die Tubenligatur**

(71) Anmelder: Karl Storz GmbH & Co. KG, 78532 Tuttlingen (DE)
(72) Erfinder: Hahn, Martin, 88605 Boll (DE)
(74) Vertreter: Weller, Wolfgang

(57) **Zusammenfassung**

Ein Ringapplikator (10) für die Tubenligatur weist ein Außenrohr (12) und ein in diesem axial verschiebbares Innenrohr (34) auf, in dem axial verschiebbar ein Greifer aufgenommen ist. Vom distalen Ende des Außenrohrs (12) steht ein distaler Endabschnitt des Innenrohrs (34) vor, auf dem Ringe aufbringbar sind. Es wird vorgeschlagen, dass der distale Endabschnitt eine solche axiale Länge aufweist, dass zumindest vier Ringe (62-65) aufbringbar sind und dass ein Schieber (26) beim Einziehen des distalen Endabschnittes des Innenrohres gegen verstellbare Anschläge (20) läuft, die in einer ersten Anschlagstellung ein Abstreifen von zwei Ringen (64,65) und in einer zweiten Anschlagstellung ein Abstreifen von zwei weiteren Ringen (62,63) erlaubt.

## Beschreibung

Die Erfindung betrifft einen Ringapplikator für die Tubenligatur, insbesondere für die laparoskopische Unterbindung des Eileiters, mit einem Außenrohr, mit einem in dem Außenrohr axial verschiebbaren Innenrohr, mit einem im Innenrohr axial verschiebbaren Greifer und mit einem auf dem Außenrohr verschiebbaren Schieber, der mit dem Greifer verbunden ist, wobei der Greifer mittels des Schiebers in das Innenrohr vollständig einziehbar ist, und wobei vom distalen Ende des Außenrohrs ein distaler Endabschnitt des Innenrohres vorsteht, auf den Ringe aufbringbar sind, wobei die Ringe beim Einziehen des distal vorstehenden Endabschnittes des Innenrohrs in das Außenrohr hinein abgestreift werden.

Unter Tubenligatur soll jede Verbindung oder auch Abschnürung von röhren- bzw. zylinderförmigem Gewebe verstanden werden.

Ein derartiger Applikator wird von der Anmelderin bspw. unter der Produktbezeichnung 26174 RA vertrieben

Ringapplikatoren werden insbesondere bei der endoskopischen Ringsterilisation eingesetzt. Dazu werden auf den distal vom Außenrohr vorstehenden Endabschnitt des Innenrohres zwei elastische Ringe aufgebracht. Dazu müssen die Ringe extrem gedehnt werden. Werden die Ringe abgestreift ziehen sie sich wieder zusammen und weisen dann eine mittlere Ringöffnung im Bruchteil von Millimetern auf.

Mit den Ringapplikatoren sollen die Ringe um eine Schleife eines röhrenförmigen Organs, meist des weiblichen Eileiters gelegt werden, um diesen so weit durch den Ring abzuquetschen, dass keine Eizellen mehr durch den Eileiter in die Gebärmutter übertreten können.

Der Ringapplikator weist dazu einen Greifer auf, der distalseitig zwei hakenartige Greiferglieder aufweist, die mit einer im Inneren des Innenrohrs aufgenommenen Zugstange verbunden sind.

Die Greiferglieder sind so geformt, dass sie beim Ausschieben aus dem distalen Ende des Innenrohrs radial aufspreizen. Beim Einziehen in das Innere des Innenrohrs werden diese radial aufeinander zu bewegt, so dass sie bspw. einen dazwischen aufgenommenen Eileiter ergreifen. Beim Einziehen des Greifers in das Innenrohr wird dann eine Schleife des von den beiden Greiferglieder ergriffenen Eileiters in das Innere des Innenrohrs eingezogen. Auf dem vom Außenrohr vorstehenden distalen Endabschnitt des Innenrohres sind zwei Ringe in dem zuvor erwähnten radial extrem aufgeweiteten Zustand aufgebracht. Wird nun in diesem Zustand, also in dem die Schleife in das Innere des Innenrohrs vom Greifer eingezogen ist, der distale Endabschnitt des Innenrohrs in das Außenrohr eingezogen, streift dieses die beiden Ringe ab und diese ziehen sich wieder zusammen und legen sich dabei um den Hals der Schlinge und quetschen dabei den Eileiter bzw. die beiden Eileiterstränge so ab, dass keine Eizellen mehr durch den Eileiter hindurchtransportiert werden können. Nach dem Abstreifen und Überbringen der Ringe auf den Hals der Schleife wird diese wieder aus dem Innenrohr durch den Greifer ausgeschoben und freigegeben.

Da eine weibliche Person zwei Eileiter besitzt wurde in den Anfangszeiten dieser Sterilisationstechnologie versucht während eines Eingriffes jeweils einen Ring auf die beiden Eileiter zu überbringen.

Dabei wurde festgestellt, dass das definierte Abstreifen von nur einem Ring nur sehr schwierig und konstruktiv sehr aufwändig durchgeführt werden kann.

So ist bspw. aus der DE 201 16 701 U1 bekannt geworden konstruktive Maßnahmen zu schaffen um die beiden Ringe nacheinander definiert abzustreifen. Dazu sind konstruktiv aufwändige Maßnahmen vorgesehen, bspw. eine Kulissen/Treppenstufenführung um das Abstreifen der beiden Ringe nacheinander durchführen zu können.

Bei der Sterilisationstechnik, bei der jeweils ein Ring pro Eileiter gesetzt wird, wurde im Laufe der Zeit festgestellt, dass es vorkommen kann, dass beim Setzen ein Ring reißen kann oder dass ein Ring nicht ausreicht um eine hundertprozentige Sterilisierung durchzuführen. Es ist ja zu bedenken, dass die Ringe extrem gedehnt werden, so dass beim Abstreifen es vorkommen kann, dass ein Ring reißt und nicht mehr zur Verfügung steht.

Daher wird nunmehr so verfahren, dass mit einem solchen Ringapplikator in einem Arbeitsvorgang zwei Ringe an eine Schleife eines Eileiters gesetzt werden.

Dazu wird zunächst der Ringapplikator mit den zwei Ringen beladen, dann wird der Ringapplikator minimal-invasiv, über einen Trokar oder eine dergleichen Vorrichtung in den Bauchraum eingeführt, es wird ein Eileiter gesucht, dieser wird mit den Greifergliedern erfasst und in einem Überbringvorgang werden beide Ringe auf die Schleife dieses Eileiters verbracht.

Der Ringapplikator wird dann vom Körper abgezogen, außerhalb des Körpers wieder mit zwei Ringen beladen, muss dann erneut in den Körper eingeführt werden und es muss der zweite noch nicht sterilisierte Eileiter lokalisiert werden und dann nochmals der selbe Vorgang durchgeführt werden, also der Eileiter ergriffen in das Innenrohr eingezogen und erneut zwei Ringe positioniert werden.

Insgesamt ist das Verfahren sehr aufwändig, der Operateur muss zum Sterilisieren einer Patientin zwei in sich abgeschlossene Eingriffe durchführen, nämlich Beladen des Applikators, Einführen in den Körper, Lokalisieren des zu sterilisierenden Eileiters, Durchführen der Sterilisation durch Überbringen der beiden Ringe und Abziehen des Instrumentes vom Körper.

Es ist daher Aufgabe der vorliegenden Erfindung hier Abhilfe zu schaffen und einen Ringapplikator zu schaffen, mit dem einfacher und in wesentlich kürzerer Eingriffszeit zwei Eileiter sterilisiert werden können.

Erfindungsgemäß wird die Aufgabe dadurch gelöst, dass der vom Außenrohr vorstehende distale Endabschnitt des Innenrohrs eine solche axiale Länge aufweist, dass zumindest vier Ringe aufbringbar sind, und dass der Schieber beim Einziehen des distalen Endabschnittes des Innenrohrs gegen einen verstellbaren Anschlag läuft, der in einer ersten Anschlagstellung ein Abstreifen von zwei Ringen während eines ersten Hubes ermöglicht, und der in zumindest eine weitere zweite Anschlagstellung bringbar ist, in der er ein Abstreifen von zwei weiteren Ringen während eines weiteren zweiten Hubes ermöglicht.

Ein erster Vorteil besteht darin, dass auf den distal vorstehenden Endabschnitts des Innenrohres zumindest vier Ringe aufgebracht werden können. Das bedeutet es reicht ein einziger Beladevorgang zum Beginn des Eingriffes vor, durch den die vier notwendigen Ringe vorhanden sind, so dass während des Eingriffes der Applikator nicht vom Körper abgezogen werden braucht.

Zumindest vier Ringe bedeutet, dass auf jeden Fall die vier Ringe die man zum Sterilisieren der beiden Eileiter benötigt aufgebracht werden, es können aber quasi als Reserve oder Vorsichtsmaßnahme auch noch ggf. zwei oder mehr weitere Ringpaare aufgebracht werden.

Dieses Aufbringen der Ringe ist ja vor dem Eingriff durchzuführen, so dass keine störende Faktoren wie Körperflüssigkeiten, Blut oder dergleichen vorhanden sein können, die ein Aufbringen und den exakten Sitz beeinflussen bzw. stören könnten. Ferner kann man den korrekten Sitz aller Ringe kontrollieren und man kann auch überprüfen, ob bei dem Aufschieben und dem damit verbundenen Dehnen schon Verformungen, Einrisse oder Verdrillungen der Ringe stattgefunden haben, die ein anschließendes exaktes Abschieben und Positionieren negativ beeinflussen oder gar verhindern könnten. Dadurch sind die Voraussetzungen für eine exakte Überbringung und Positionierung der Ringe als Ringpaare günstig und es wird auch gleichzeitig schon die Zeitersparnis dahingehend erzielt, dass der Applikator nicht mehr vom Körper abgezogen und neu mit Ringen bestückt werden muss.

Durch Vorsehen des verstellbaren Anschlages kann sichergestellt werden, dass in einem ersten Hub genau zwei Ringe abgestreift werden, d.h. nicht mehr und nicht weniger. Die Handhabungsperson kann somit den Anschlag in eine bestimmte gewünschte Position verbringen die sicherstellt, dass in einem ersten Hub gerade die ersten zwei aufgeschobenen Ringe wieder abgestreift und auf die Schleife des in das Innenrohr eingezogenen Eileiters verbracht werden können.

Nach Freigeben der Schleife mit den bereits daran angebrachten beiden Ringen kann der Applikator im Körper verbleiben und der Operateur braucht nunmehr nur noch den zweiten Eileiter anzuzielen und mit dem Greifer ergreifen.

Der Anschlag kann nunmehr in eine zweite Anschlagstellung bewegt werden, durch die sichergestellt ist, dass die nächsten zwei Ringe definiert abgestreift und auf den Eileiter überbracht werden können. Somit können während eines einzigen Eingriffs jeweils zwei Ringe an zwei verschiedene Eileiter einfach und sicher überbracht werden.

Falls vorgesehen ist, dass noch ein weiterer Ring oder weitere Ringpaare aufgenommen sind, könnten in demselben Eingriff noch weitere Ringe überbracht werden, bspw. wenn festgestellt werden sollte, dass einer der beiden gesetzten Ringe gerissen ist und sich gelöst hat oder die Ringe aufgrund anatomischer Gegebenheit nicht ausreichend fest sitzen um den Eileiter abzuquetschen.

Dies wird in aller Regel nur als Redundanz dienen, da im Regelfall das Setzen von zwei Ringen eine ausreichende Sicherheit dahingehend gibt, dass der Eileiter so abgequetscht ist, dass keine Eizellen mehr durchbewegt werden können.

Der verstellbare Anschlag schafft ganz definierte Hubstrecken über die das Innenrohr in das Außenrohr eingezogen wird, wodurch sichergestellt ist, dass die gewünschte Anzahl von Ringen abgestreift werden, also bspw. zwei. Dadurch ist dann auch ausgeschlossen, dass versehentlich nur einer oder ggf. drei Ringe gleichzeitig abgestreift werden.

Daher ist der Eingriff nicht nur einfach und in kurzer Zeitdauer, sondern auch mit einer sehr hohen Erfolgsquote durchführbar.

In einer weiteren Ausgestaltung der Erfindung ist der Anschlag an einem Drehelement am proximalen Ende des Ringapplikators angeordnet, und erlaubt in unterschiedlichen Drehstellungen den ersten, den zweiten bzw. weitere Hübe.

Diese Maßnahme hat den Vorteil, dass der Arzt definiert und bewusst durch Einstellen des Drehelements die entsprechenden Hübe möglich machen kann, so dass er die Paare an Ringen nach und nach abstreifen bzw. an den Hals der ins Innenrohr eingezogenen Schleife des Eileiters legen kann.

In einer weiteren Ausgestaltung der Erfindung sind an dem Drehelement Merkmale angebracht, die dem Benutzer anzeigen, welcher Hub des Schiebers durchführbar ist.

Diese Maßnahme hat den Vorteil, dass der Benutzer ganz bewusst den Hub einstellen kann, den er benötigt oder der für ihn günstig ist. So kann ihm angezeigt werden, dass in einer bestimmten Stellung nur die ersten beiden Ringe überbracht werden können, nachdem er den ersten Eileiter ergriffen und in das Innenrohr eingezogen hat.

Dadurch ist ausgeschlossen, dass er einen anderen Hub ausführt. Er kann sich anschließend darauf konzentrieren den anderen Eileiter zu suchen, zu lokalisieren und einziehen und kann dann den Anschlag in eine solche Stellung bringen, die ihm nun den zweiten oder weiteren Hub erlaubt, um nun definiert und fest und sicher sitzend das nächste Paar an Ringen an den nächsten Eileiter zu überbringen.

In einer weiteren Ausgestaltung der Erfindung stehen vom Drehelement nach distal Nocken vor, die ja nach Drehstellung des Drehelements den ersten, den zweiten oder ggf. weitere Hübe des Schiebers erlauben bzw. begrenzen.

Diese Ausgestaltung stellt konstruktiv einfache, dennoch mit hoher Maßgenauigkeit herstellbare Maßnahmen bereit, um die definierten Hübe sicher zu stellen. Zusätzlich stellen die vorspringenden Nocken für die Bedienungsperson noch ein Orientierungsmerkmal dar, aus dem man ableiten kann, welcher Hub möglich ist und welcher nicht. Diese Nocken können das zuvor erwähnte Orientierungsmerkmal selbst sein, oder können auch zusätzlich zu diesem vorhanden sein. Derartige Nocken sind fertigungstechnisch einfach zu bewerkstelligen und stellen auch Bauelemente dar, die einfach zu reinigen sind. Es sind keine kostenaufwändige und technisch komplizierte möglicherweise viele Bakteriennischen aufweisende Steuerkurven notwendig.

In einer weiteren Ausgestaltung der Erfindung ist die Bemaßung von Innenrohr und Außenrohr eine funktionsgerechte Bemaßung, wodurch ein Abstreifen von jeweils zwei Ringen pro Hub sicherstellbar ist.

Diese technische Maßnahme hat den Vorteil, dass schon bei der originären Herstellung dieser beiden Teile ein Beitrag dazu geleistet wird das exakte Abschieben von jeweils zwei Ringen pro Hub sicher zu stellen.

Bei dem eingangs erwähnten Ringapplikator der Anmelderin war nur eine sogenannte fertigungsgerechte Bemaßung durchzuführen, da ja lediglich sichergestellt sein musste, dass die zwei aufgebrachten Ringe auf jeden Fall vollständig abgestreift werden konnten. Daher waren relativ große Toleranzen möglich, die sich ggf. zu einem sehr großen Toleranzfeld aufaddiert haben, das schon im Bereich der Breite eines Ringes war. Mit einer solchen fertigungsgerechten Bemaßung müssten ggf. erhebliche Nach- oder Justierarbeiten durchgeführt werden, um ein klar definiertes stufenweises Abstreifen von jeweils zwei Ringen sicherzustellen.

Die nunmehr funktionsgerechte Bemaßung mit geringeren Toleranzen, die aber nicht zu eng sein müssen, wird ein Beitrag dazu geleistet, solche Ringapplikatoren als Großserienprodukte herzustellen und dennoch sicherzustellen, dass pro Hub die gewünschte Zahl an Ringen abgestreift werden kann.

So wurden bspw. bei den eingangs erwähnten Ringapplikatoren mit Toleranzen von ± 0,35 mm gearbeitet woraus sich ein Toleranzfeld von 0,7 mm ergibt. Bei Toleranzen am Innenschaft von ± 0,3 mm entstand ein Toleranzfeld von 0,6 mm, so dass aufaddiert Toleranzen vorhanden waren, die schon etwa der Breite eines Ringes entsprechen.

Durch die nunmehr vorgeschlagene Umstellung auf eine funktionsgerechte Bemaßung, die immer noch gewisse Toleranzen erlauben, bspw. ± 0,1 mm, werden Voraussetzungen geschaffen, um die definierten Hub- und somit Abstreifbewegungen zu ermöglichen. Diese Toleranzen sind auch in Großserienprodukten ohne allzu großem Produktionsaufwand einzuhalten. Im Zusammenwirken bspw. mit den Nocken die vom Drehelement nach distal vorstehen, sind durch technisch einfache Maßnahmen exakte Hübe zu bewerkstelligen, die das sichere Überbringen der jeweils zwei Ringe sicherstellen.

In einer weiteren Ausgestaltung der Erfindung weist das Innenrohr einen axialen Längsschlitz auf, durch den ein radialer Stift des Schiebers reicht, der mit einer im Innenrohr aufgenommenen Zugstange des Greifers verbunden ist.

Diese an sich bekannte Maßnahme hat den Vorteil, dass konstruktiv einfach und wenig raumergreifend die Bewegungen des Greifers, also das Aus- und Einfahren in das Innenrohr, durchgeführt werden können.

In einer weiteren Ausgestaltung der Erfindung kommt der axiale Längsschlitz des Innenrohres auf Höhe eines axialen Längsschlitzes des Außenrohres zum Liegen, der länger ausgebildet ist als der axiale Längsschlitz des Innenrohres.

Diese ebenfalls an sich bekannte Maßnahme hat zusammen mit den neu vorgeschlagenen Maßnahmen den Vorteil, dass die Einziehbewegung des Innenrohres in das Außenrohr, bei bereits eingezogenem Greifer mit wenig raumergreifenden und technisch einfachen Maßnahmen durchführbar ist. Der Stift des längs des Außenrohr verschiebbaren Schiebers ist ja mit dem Greifer verbunden und steuert somit das Ein- und Ausschieben des Greifers ins Innenrohr und kann auch dann zusätzlich zum Bewegen des Innenrohrs relativ zum Außenrohr beim Einziehen des Innenrohres herangezogen werden. Ein solcher Stift bzw. die Langlochöffnungen können durch einfache Maßnahmen maßhaltig hergestellt werden und sind auch einfach zu reinigen und zu sterilisieren und stellen keine komplizierten Bauteile dar, die Bakteriennischen bieten.

In einer weiteren Ausgestaltung der Erfindung verlaufen die Hubbewegungen des Schiebers zum Einziehen des Innenrohrs gegen die Kraft von zumindest einer Feder.

Diese Maßnahme hat verschiedene Vorteile. Ein Vorteil besteht darin, dass der Greifer mit einem bestimmten für den Benutzer erkennbaren Kraftaufwand relativ zum Innenrohr bewegt werden, ohne dass dieses seine Relativposition zum Außenrohr verändert. Erst wenn dieses gegen die Kraft der Feder bewegt wird, kann das Innenrohr ins Außenrohr eingezogen werden und die Ringe abgestreift werden.

Beim Freigeben des Schiebers wird das Innenrohr wieder in eine definierte Position relativ zum Außenrohr gedrückt.

Diese Federkraft gibt der Handhabungsperson das Gefühl, ob er eine Bewegung ohne Gegenkraft durchführt, die dazu dient, um den Greifer in das Innenrohr einzuziehen bzw. das Gefühl, da er nunmehr die Kraft einer Feder überwinden muss, dass er sich in einer der stufenweisen Hubbewegungen zum Einziehen des Innenrohres und dabei Abstreifen der Ringe befindet.

Diese Maßnahme trägt somit zur Erhöhung der Bediensicherheit bei.

In einer weiteren Ausgestaltung der Erfindung ruht das proximale Ende des Innenrohrs auf einer Feder, die sich auf einem proximalseitigen Gehäuse abstützt.

Diese Maßnahme hat den Vorteil, dass sich die zuvor erwähnten Maßnahmen der Abfederung der Hubvorgänge zur Außenseite geschützt in dem Gehäuse abspielen und auch dass relativ wenige zusätzliche Bauteile dazu notwendig sind, da sich die Feder einerseits auf dem Innenrohr und andererseits an der Innenseite des Gehäuses abstützt.

Es versteht sich, dass die vorstehend genannten und die nachstehend noch zu erläuternden Merkmale nicht nur in den angegebenen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels näher beschrieben und erläutert. Es zeigen:
- Fig. 1: eine perspektivische Darstellung eines Ringapplikators,
- Fig. 2: eine Seitenansicht des Ringapplikators von Fig. 1,
- Fig. 3: eine stark vergrößerte, teilweise geschnittene distale Endansicht des Ringapplikators von Fig. 1 und 2,
- Fig. 4: eine der Fig. 3 vergleichbare Stellung bei in das Innenrohr eingezogenen Greifergliedern,
- Fig. 5a: eine Situation beim Beladen des distal vorstehenden Endabschnittes des Ringapplikators mit Ringen mittels Hilfsgeräten, einen Querschnitt eines ungespreizten Ringes,
- Fig. 5b: einen Querschnitt eines gespreizten Ringes,
- Fig. 6: eine der Darstellung von Fig. 4 vergleichbare Darstellung des distalen Endbereiches des Applikators, wobei vier Ringe auf dem distalen Endabschnitt aufgebracht sind,
- Fig. 7: eine Situation während eines Eingriffes, wobei sich die Greiferglieder einem Eileiter, der sterilisiert werden soll, nähern,
- Fig.8: eine Situation, nachdem die Greifer einen Abschnitt des Eileiters ergriffen und eine Schleife des Eileiters in das Innere des Innenrohrs eingezogen haben,
- Fig. 9: eine Situation, in der der distale Endabschnitt des Innenrohrs um einen ersten Hub in das Außenrohr hineinbewegt worden ist, wobei die ersten zwei Ringe abgestreift und auf einen Hals der Schleife des Eileiters bereits überbracht worden sind, die beiden anderen Ringe aber noch auf dem distalen Endabschnitt sitzen,
- Fig. 10: eine Situation nach der Freigabe der mit den beiden Ringen versehenen Schleife durch die Greiferglieder und
- Fig. 11: eine Situation, bei der ein anderer Eileiter ergriffen und in das Innenrohr eingezogen worden ist, das Innenrohr vollständig in das Außenrohr eingezogen wurde, so dass auch die restlichen zwei Ringe auf die Schlaufe des weiteren Eileiters überbracht worden sind.

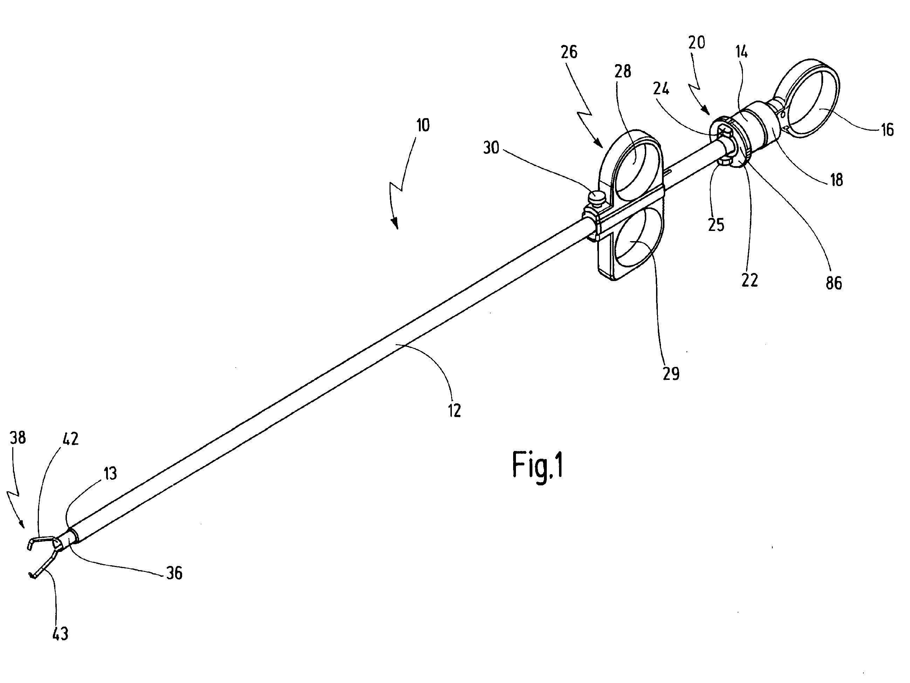

Ein in den Fig. 1 bis 11 dargestellter Ringapplikator ist in seiner Gesamtheit mit der Bezugsziffer 10 bezeichnet.

Der Ringapplikator 10 weist ein Außenrohr 12 auf, dessen proximales Ende mit einem Gehäuse 14 verbunden ist. Vom Gehäuse 14 steht proximalseitig ein Daumenring 16 ab, der über eine Kappe 18 mit dem Gehäuse 14 verschraubt ist. Der Daumenring 16 ist frei drehbar.

Distalseitig am Gehäuse 14 ist ein Anschlag 20 angeordnet.

Der Anschlag 20 weist ein ringförmiges Drehelement 22 auf, das um die Mittellängsachse bzw. um das Außenrohr 12 drehbar ist. Vom Drehelement 22 stehen distalseitig zwei Nocken 24 und 25 vor, die diametral gegenüber liegen.

Auf der Außenseite des Außenrohres 12 ist ein Schieber 26 axial verschiebbar aufgenommen, der zwei diametral gegenüberliegende Fingerösen 28 und 29 aufweist. Ein proximalseitiges Ende des Schiebers 26 stößt beim Verschieben des Schiebers 26 nach proximal auf den Anschlag 20.

In der in Fig. 1 dargestellten Drehstellung des Anschlages 20 bzw. des Drehelements 22 stößt der Schieber 26 auf die beiden Nocken 24 und 25. Wie dies später noch näher erläutert wird, bilden diese einen Anschlag, der einen ersten Hub begrenzt. Wird das Drehelement 22, das in dieser Stellung über eine Kugelrastsicherung drehfest verrastet ist, um 90° verdreht, können die Nocken 24 und 25 nicht mehr mit dem proximalen Ende des Schiebers 26 in Berührung treten. Dieser kann dann um die axiale Länge der Nocken 24, 25 weiter nach proximal verschoben werden, bis er an eine distalseitige ringförmige Stirnfläche 86 des Drehelementes 22 fährt, die somit einen weiteren Anschlag zur Begrenzung eines weiteren zweiten Hubs darstellt.

Im Außenrohr 12 ist ein Innenrohr 34 aufgenommen, dessen Außendurchmesser etwa dem lichten Innendurchmesser des Außenrohrs 12 entspricht.

Ein distaler Endabschnitt 36 ragt dabei über das distale Ende 30 des Außenrohrs 12 hinaus.

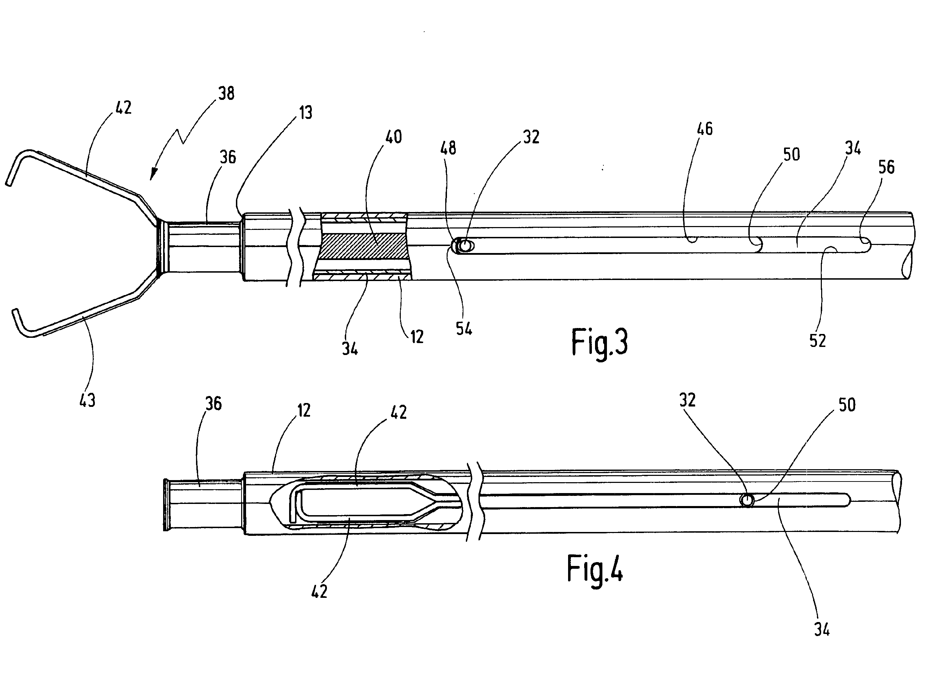

Im Innenrohr 34 ist ein Greifer 38 aufgenommen (wie insbesondere aus Fig. 3 und 4 ersichtlich) der eine im Innern des Innenrohrs 34 verlaufende Zugstange 40 aufweist, die proximalseitig in zwei Greifergliedern 42 und 43 endet. Die Greiferglieder 42 und 43 sind so vorgebogen bzw. vorgespannt, dass sie sich, wenn sie aus dem Innenrohr 34 ausgetreten sind (Fig. 3) radial spreizen.

Wird die Zugstange 40 nach proximal bewegt, bewegen sich die beiden Greiferglieder 42 und 43 radial aufeinander zu und können vollständig in das Innenrohr 34 eingezogen werden, wie das in Fig. 4 dargestellt ist. Daraus ist ersichtlich, dass die distalen abgebogenen Enden der Greiferglieder 42, 43 einander übergreifen.

Wie insbesondere aus Fig. 3 und 4 zu entnehmen, ist im Innenrohr 34 ein axialer Längsschlitz 46 vorhanden, der ein distales Ende 48 und ein proximales Ende 50 zeigt.

Der axiale Längsschlitz 46 des Innenrohres 34 liegt im Bereich eines axialen Längsschlitzes 52 im Außenrohr 12.

Das distale Ende 54 des axialen Längsschlitzes 52 liegt etwa auf Höhe des distalen Endes 48 des axialen Längsschlitzes 46 im Innenrohr 34.

Der axiale Längsschlitz 52 ist länger ausgebildet als der axiale Längsschlitz 46. Demzufolge kommt dessen proximales Ende 56 weiter proximal zu liegen als das proximale Ende 50 des axialen Längsschlitzes 46 des Innenrohrs 34.

Aus Fig. 1 ist zu entnehmen, dass der Schieber 26 einen radial verlaufenden Schraubstift 30 trägt, dessen radialer Stift 32 (s. Fig. 3 und 4) sowohl durch den axialen Längsschlitz 52 des Außenrohres 12 als auch durch den axialen Längsschlitz 46 des Innenrohres 34 reicht und mit der Zugstange 40 fest verbunden ist.

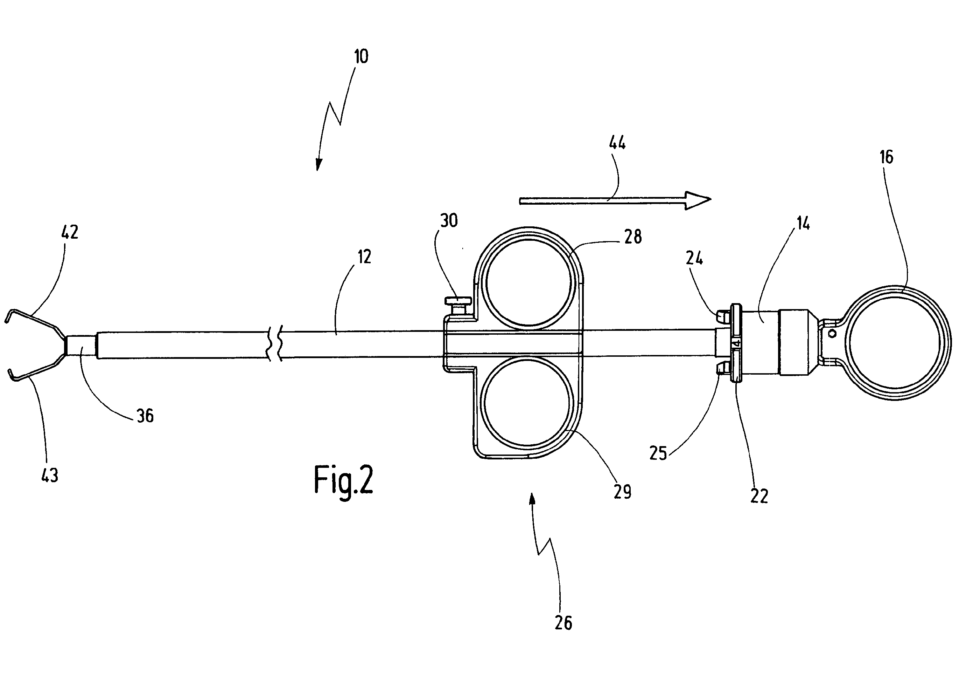

Die Position des Schiebers 26 von Fig. 1 und 2 entspricht der Position von Fig. 3, d.h. die Greiferglieder 42 und 43 sind vollständig auf dem Innenrohr 34 ausgefahren und maximal radial gespreizt. In dieser Stellung kommt der Schieber 26 bzw. dessen Stift 32 in einer Position zu liegen, in der er am distalen Ende 48 des axialen Längsschlitzes 46 im Innenrohr 34 zu liegen kommt, der in etwa auch dem distalen Ende 54 des axialen Längsschlitzes 53 im Außenrohr 12 entspricht.

Wird der Schieber 26 aus dieser Position nach proximal bewegt, wie das in Fig. 2 durch einen Pfeil 44 angedeutet ist, wird der Greifer 38 nach proximal bewegt, wobei sich die Greiferglieder 42 und 43 radial aufeinander zu bewegen und in das Innere vollständig hineingezogen werden, wie das in Fig. 4 dargestellt ist. In dieser Position kommt der Stift 32 am proximalen Ende 50 des axialen Längsschlitzes 46 des Innenrohrs 34 zu liegen.

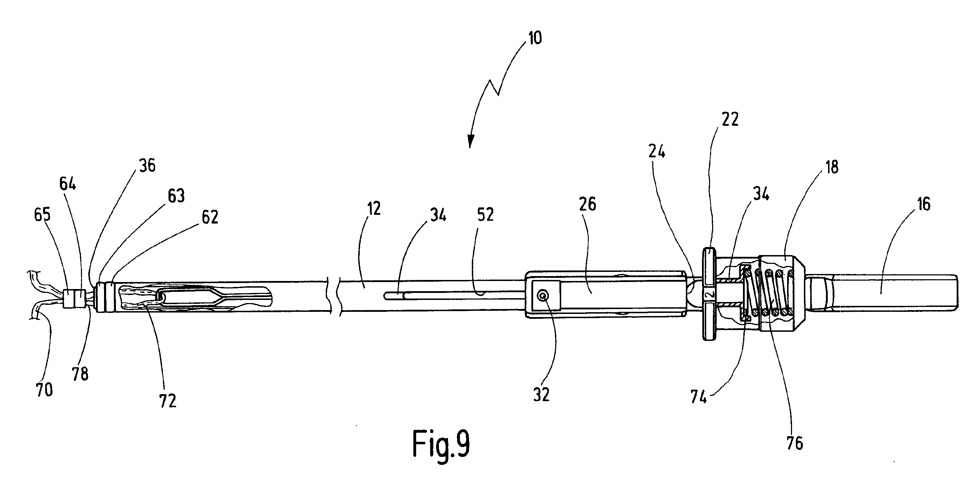

Aus dieser Stellung kann der Schieber 26 bzw. dessen Stift 32 nur unter Überwindung der Kraft einer Feder, wie sie später in Zusammenhang mit Fig. 9 beschrieben wird, weiter nach proximal bewegt werden. Dabei würde der Stift 32 auch das Innenrohr 34 nach proximal ziehen, wobei dann der distale Endabschnitt 36 nach und nach in das Außenrohr 12 eingezogen wird.

Dieser Bewegungsablauf und die entsprechende Steuerung soll nachfolgend beschrieben werden.

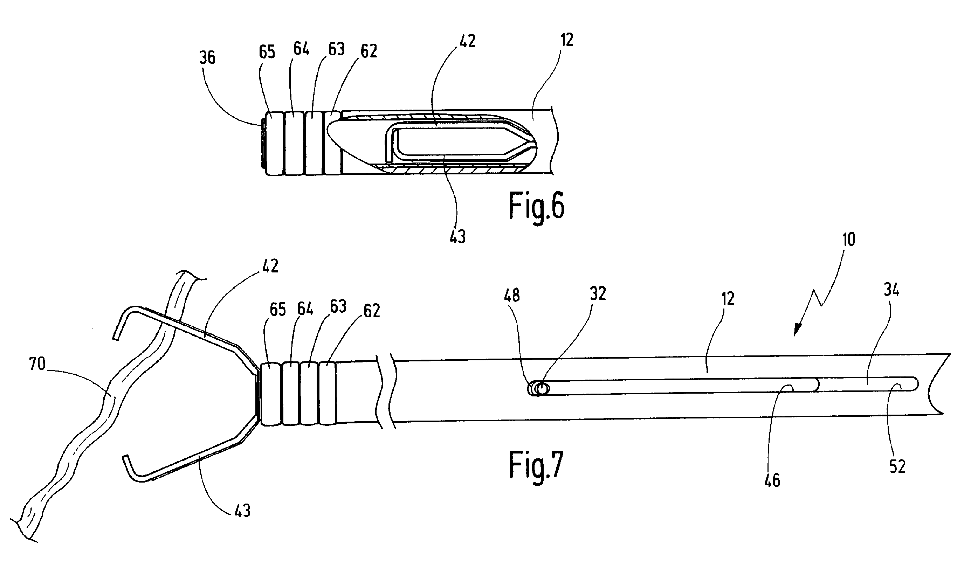

Der aus Fig. 4 ersichtliche vom Außenrohr 12 vorspringende distale Endabschnitt 36 des Innenrohres 34 dient dazu, dass auf diesen vier Ringe 62, 63, 64, 65 aufgebracht werden, wie die in Fig. 6 dargestellt ist. Es ist ersichtlich, dass die axiale Länge des distalen Endabschnitts 36 gerade ausreicht, dass die vier Ringe 62 bis 65 eng aneinanderliegend aufgenommen werden können.

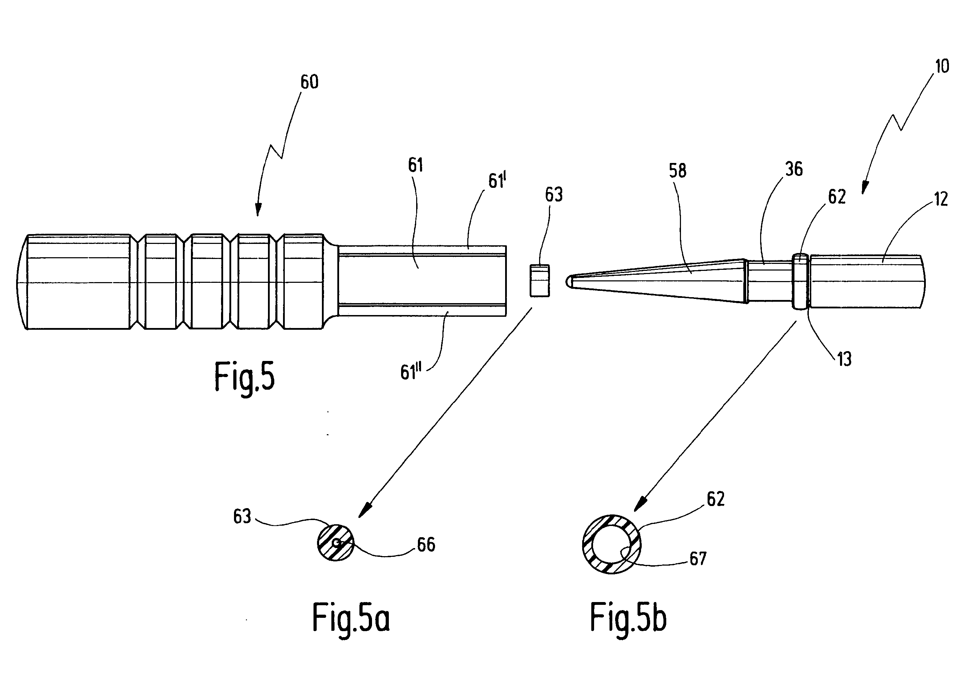

Wie dies erfolgt, soll anhand von Fig. 5 bzw. Fig. 5a bzw. 5b erläutert werden.

Dazu wird, wie das aus Fig. 5 auf der rechten Seite ersichtlich ist, auf den distalen Endabschnitt 36 ein Konus 58 aufgesetzt. Das distale spitze Ende des Konus 58 ist so, dass daran ein nicht gespreizter Ring 63 angesetzt werden kann.

Wie aus dem Schnitt von Fig. 5a ersichtlich, besteht der Ring 63 aus einem elastischen Material mit einer relativ dicken Ringwand und einer äußerst geringen mittigen Öffnung 66. Der Ring 63 besteht aus einem elastischen Kunststoffmaterial, beispielsweise einem elastischen Silikonmaterial. Der Durchmesser beträgt etwa 3,6 mm, die Breite 2 mm, der Durchmesser der Öffnung beträgt etwa 0,5 mm. Ein auf die Spitze des Konus 58 angesetzter Ring 63 kann mittels eines Ringaufschiebers 60 über den Konus 58 auf den distalen Endabschnitt 36 geschoben werden.

Dazu weist der Ringaufschieber 60 vier durch Längsschlitze voneinander geteilte Spreizkeile 61, 61', 61" auf. In Fig. 5 ist auf der rechten Seite dargestellt, wie bereits ein Ring 62 auf den distalen Endabschnitt 36 aufgeschoben ist, und zwar so weit, dass er an dem distalen Ende 13 bzw. an der distalen Ringfläche des Außenrohrs 12 anliegt.

Aus der Schnittdarstellung von Fig. 5b ist ersichtlich, dass dadurch der Ring 62 erheblich gespreizt wurde, insbesondere wurde der Durchmesser der mittigen Öffnung 67 erheblich gespreizt, nämlich auf das Maß des distalen Endabschnittes 36.

Aufgrund der Elastizität, die dazu führt, dass ein gespreizter Ring wieder in die in Fig. 5a dargestellte Position zurückkehren will, sitzt dieser zum einen sehr fest auf dem distalen Endabschnitt 36. Diese erhebliche Rückstellkraft führt aber auch dazu, dass ein gewisser Kraftaufwand notwendig ist, um einen solchen Ring wieder abzuschieben.

Nach und nach werden vier Ringe 62, 63, 64, 65 aufgeschoben. Anschließend wird der Konus 58 wieder abgenommen. Dieser Vorgang wird zur Vorbereitung eines Sterilisationseingriffs durchgeführt, d.h. die vier Ringe 62 bis 64 werden wie in Fig. 6 dargestellt aufmunitioniert.

In dieser Position wird nun der Ringapplikator 10 minimal-invasiv, beispielsweise über einen entsprechenden Trokar, in eine Gebärmutter einer Patientin eingeführt.

Dieser Vorgang kann entweder durch ein zusätzliches Endoskop oder dadurch, dass der Ringapplikator in ein mit einer Optik versehenes Instrument in die Gebärmutter eingebracht wird, beobachtet werden.

Nunmehr wird der Schieber 26 nach distal verschoben, so dass die Greiferglieder 42 und 43 wieder vollständig aus dem Innenrohr 34 ausfahren und sich radial spreizen, wie das in Fig. 7 dargestellt ist. Es wird ein rohrförmiges Organ, wie ein Eileiter 70, anvisiert und dieser wird zwischen die Greiferglieder 42 und 43 gebracht. Beim Einziehen des Greifers 38 in das Innere des Innenrohres 34 wird ein Abschnitt des Eileiters 70 in Form einer Schleife 72 eingezogen.

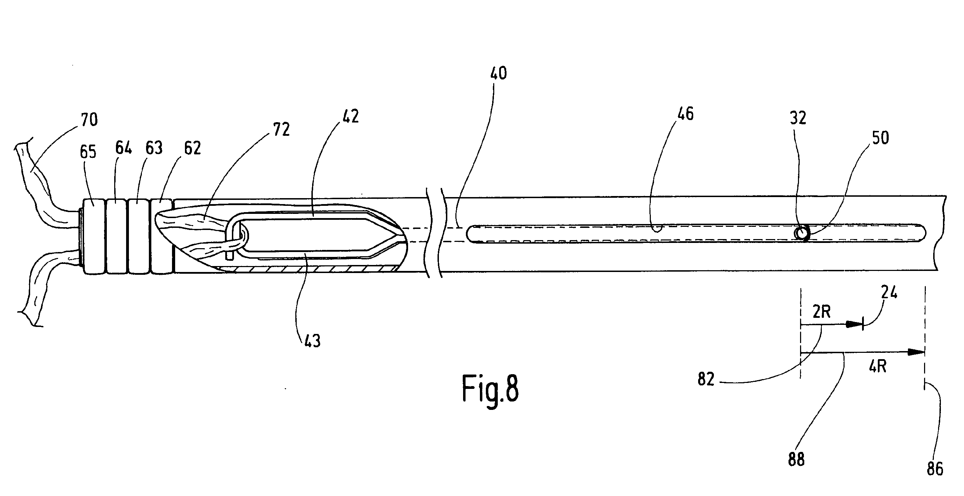

Diese Situation ist in Fig. 8 dargestellt. Daraus ist ersichtlich, dass die beiden Greiferglieder 42 und 43 geschlossen sind und nunmehr eine Schleife 72 des Eileiters 70 in das Innere des Innenrohrs 34 eingezogen haben. Wie zuvor bereits in Zusammenhang mit Fig. 4 beschrieben, kommt in dieser Position der Stift 32 am proximalen Ende 50 des axialen Längsschlitzes 46 des Innenrohres 34 zu liegen.

In dieser Position wird das Drehelement 22 des Anschlages 20 in eine solche Position gebracht, wie es in Fig. 1 und 2 dargestellt ist, d.h. die axial vorstehenden Nocken 24, 25 sind so ausgerichtet, dass sie eine Bewegung des Schiebers 26 nach proximal begrenzen. Wie in Fig. 8 dargestellt, kann in dieser Position der Schieber 26 um einen ersten Hub 82 weiter nach proximal verschoben werden, bis dieser die Nocken 24 bzw. 25 trifft.

Dieser erste Hub 82 entspricht der axialen Länge von zwei Ringen, also 2 R.

Es wird also der distale Endabschnitt 36 um den Hub 82 nach proximal bewegt und in das Außenrohr 12 eingezogen, dabei werden alle vier Ringe 62-65 nach distal längs des Endabschnittes 36 verschoben, jedoch werden nur die beiden vorderen Ringe 64 und 65 abgestreift.

Aufgrund der Elastizität ziehen sie sich wieder zusammen (Übergang von Fig. 5b zu Fig. 5a) und umschließen einen Hals 78 der Schleife 42 des Eileiters 70.

Diese Situation ist in Fig. 9 dargestellt.

In Fig. 9 ist auch dargestellt, dass das Innenrohr 34 proximalseitig mit einem Flansch 74 versehen ist, auf den sich eine Feder 76 abstützt, die gegenüberliegend auf der Innenseite der Kappe 18 des Gehäuses 14 abgestützt ist.

Die Federgeometrie ist so gewählt, dass das Innenrohr 34 während des Hubes 82 gegen die Kraft der Feder 76 bewegt werden muss. Dies ist so weit möglich, bis der Schieber 26 gegen die Nocke 24 des Anschlages läuft.

Dadurch ist sichergestellt, dass nur die ersten beiden Ringe 64 und 65 vom distalen Endabschnitt 36 abgestreift werden und nicht mehr und nicht weniger.

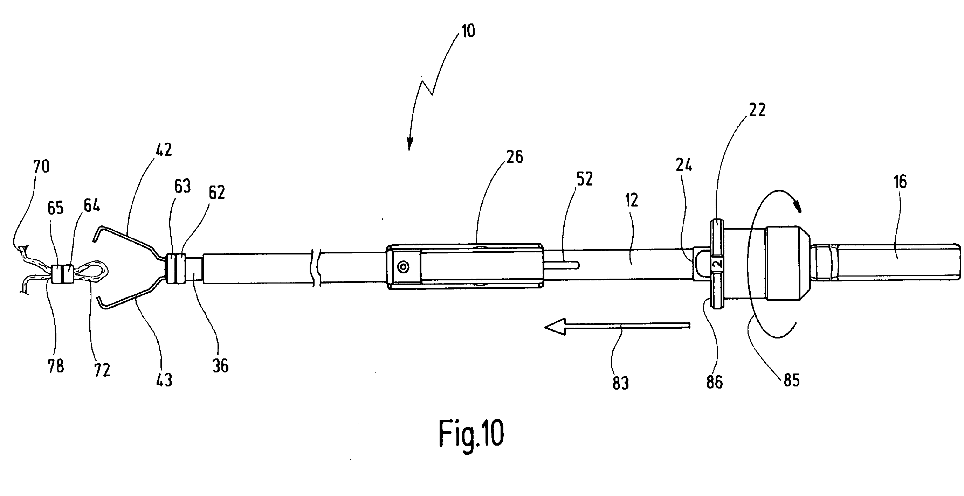

Im nächsten Schritt wird der Schieber 26 nach distal bewegt, wie das in Fig. 10 durch ein Pfeil 83 angedeutet ist.

Die beiden Greiferglieder 42 und 43 des Greifers 38 fahren vollständig aus dem Innenrohr 34 aus und geben somit den Eileiter 70 samt seiner Schleife 72, um deren Hals 78 eng anliegend die beiden Ringe 64 und 65 liegen, wieder frei.

Dadurch dass, wie zuvor beschrieben, das Innenrohr 34 gegen die Feder 76 gelagert ist, wurde dieses ebenfalls wieder so weit aus dem Außenrohr 12 geschoben, dass es über seinen gesamten distalen Endabschnitt 36 wieder vorsteht.

Die beiden Ringe 62 und 63 wurden bereits um den Hub 82 nach distal längs des distalen Endabschnittes 36 verschoben, sitzen aber nach wie vor definiert und auch beide auf dem distalen Endabschnitt 36 des Innenrohres 34.

Wie in Fig. 10 durch einen Pfeil 85 dargestellt, wird nun das Drehelement 22 um 90° verdreht. Dadurch ist möglich, dass in dieser Stellung der Schieber 26, nachdem der Greifer 38 einen weiteren Eileiter ergriffen hat und in das Innenrohr 34 eingezogen hat, um einen größeren Hub 88 bewegt werden kann, der den axialen Längenabschnitten von vier Ringen entspricht, wie das in Fig. 8 dargestellt ist. ( Hub 88 = 4 R)

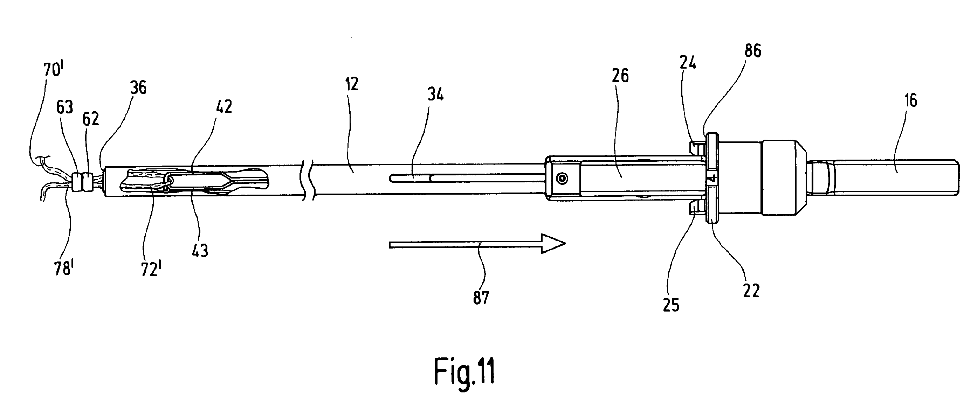

In anderen Worten ausgedrückt, kann in dieser Position der Schieber 26 bis an die distale Stirnfläche 86 des Drehelementes 22 herangefahren werden, wie das in Fig. 11 dargestellt ist.

Die Nocken 24 und 25 stören daher diesen Bewegungsablauf nicht.

Bei dieser Bewegung über den zweiten Hub 88 wird der distale Endabschnitt 36 des Innenrohres 34 vollständig in das Außenrohr 12 eingezogen, so dass auch noch die beiden auf diesem Abschnitt verbliebenen Ring 62 und 63 (s. Fig. 10) auf einen Hals 68' des weiteren Eileiters 70' verschoben werden, wie das in Fig. 11 dargestellt ist.

Aus Fig. 11 ist zu entnehmen, dass an dem Drehelement 22 ein entsprechendes Orientierungsmerkmal, hier die Zahl "4" angebracht ist, um der Benutzungsperson auch visuell anzuzeigen, dass in dieser Drehstellung der Hub 88, also einem Maß entsprechend von vier Ringen möglich ist.

Ein entsprechender Hinweis ist auch in der in Fig. 9 und 10 ersichtlichen Drehstellung des Drehelements 23 vorhanden, die es durch die Zahl "2" dem Operateur anzeigt, dass hier ein Hub 82 möglich ist, über den zwei Ringe abgestreift werden können und nicht mehr.

Aus der Position von Fig. 11 wird anschließend der Schieber 26 nach proximal verschoben, so dass der Greifer wieder aus dem Innenrohr ausgefahren wird und die Schleife 72' freigibt, wie das entsprechend in Fig. 10 dargestellt ist.

Im dargestellten Ausführungsbeispiel ist die Konstruktion so gewählt, dass in zwei aufeinanderfolgenden Hubabschnitten 82 und 88 jeweils zwei Ringe abgeschoben werden können.

Es ist einleuchtend, dass der distale Endabschnitt 36 auch länger ausgebildet sein kann, so dass ggf. als Sicherheitsmaßnahme noch weitere Ringe, bspw. weitere zwei Ringe aufgeschoben werden können. Dazu ist dann der Anschlag 20 bzw. die Langlochgeometrie so auszugestalten, dass nach Durchschreiten von erstem Hub 82 und zweitem Hub 88 noch definiert ggf. ein weiterer oder weitere Hübe möglich sind um auch diese weiteren aufgebrachten Ringe definiert abschieben zu können.

## Patentansprüche

1. Ringapplikator für die Tubenligatur, mit einem Außenrohr (12), mit einem in dem Außenrohr (12) axial verschiebbaren Innenrohr (34), mit einem im Innenrohr (34) axial verschiebbaren Greifer (38), und mit einem auf dem Außenrohr (12) verschiebbaren Schieber (26), der mit dem Greifer (38) verbunden ist, wobei der Greifer (38) mittels des Schiebers (36) in das Innenrohr (34) vollständig einziehbar ist, und wobei vom distalen Ende (13) des Außenrohrs (12) ein distaler Endabschnitt (36) des Innenrohrs (34) vorsteht, auf den Ringe aufbringbar sind, wobei die Ringe, beim Einziehen des distal vorstehenden Endabschnittes (36) des Innenrohres (34) mittels des Schiebers (26) in das Außenrohr (12), abgestreift werden, **dadurch gekennzeichnet, dass** der distale Endabschnitt (36) eine solche axiale Länge aufweist, dass zumindest vier Ringe (62-65) aufbringbar sind, und dass der Schieber (26) beim Einziehen des distalen Endabschnittes (34) des Innenrohres (34) gegen einen verstellbaren Anschlag (20) läuft, der in einer ersten Anschlagstellung ein Abstreifen von zwei Ringen (64, 65) während eines ersten Hubes (82) ermöglicht, und in zumindest einer weiteren zweiten Anschlagstellung ein Abstreifen von zwei weiteren Ringen (62, 63) während eines zweiten Hubes (88) ermöglicht.

2. Ringapplikator nach Anspruch 1, **dadurch gekennzeichnet, dass** der Anschlag (20) an einem Drehelement (22) am proximalen Ende des Ringapplikators (10) angeordnet ist, und in unterschiedlichen Drehstellungen den ersten (82) bzw. den zweiten Hub (88) begrenzt.

3. Ringapplikator nach Anspruch 2, **dadurch gekennzeichnet, dass** dem Drehelement (22) Merkmale (80) angebracht sind, die dem Benutzer anzeigen, welcher Hub (82 oder 88) des Schiebers (26) durchführbar ist.

4. Ringapplikator nach Anspruch 2 oder 3, **dadurch gekennzeichnet, dass** vom Drehelement (22) nach distal Nocken (24, 25) vorspringen, die, je nach Drehstellung des Drehelements (22) den ersten Hub (82) oder den zweiten Hub (88) oder ggf. weitere Hübe des Schiebers (26) begrenzen.

5. Ringapplikator nach einem der Ansprüche 1 bis 4, **dadurch gekennzeichnet, dass** die Bemaßung von Innenrohr (34) und Außenrohr (12) als eine funktionsgerechte Bemaßung ausgebildet ist, wodurch ein Abstreifen von jeweils zwei Ringen (64, 65 bzw. 63, 62) pro Hub (82 bzw. 88) sicherstellbar ist.

6. Ringapplikator nach einem der Ansprüche 1 bis 5, **dadurch gekennzeichnet, dass** das Innenrohr (34) einen axialen Längsschlitz (46) aufweist, durch den ein radialer Stift (32) des Schiebers (26) reicht, der mit einer im Innenrohr (34) aufgenommenen Zugstange (40) des Greifers (38) verbunden ist.

7. Ringapplikator nach Anspruch 6, **dadurch gekennzeichnet, dass** der axiale Längsschlitz (46) des Innenrohrs (34) auf Höhe eines axialen Längsschlitzes (52) des Außenrohres (12) zum Liegen kommt, der länger ausgebildet ist als der axiale Längsschlitz (46) des Innenrohres (34).

8. Ringapplikator nach einem der Ansprüche 1 bis 7, **dadurch gekennzeichnet, dass** die Hubbewegungen (82, 88) des Schiebers (26) zum Einziehen des distalen Endabschnittes (34) des Innenrohres gegen die Kraft von zumindest einer Feder (76) verlaufen.

9. Ringapplikator nach Anspruch 8, **dadurch gekennzeichnet, dass** das proximale Ende des Innenrohres (64) auf der Feder (76) ruht, die sich in einem proximalseitigen Gehäuse (14) abstützt.
